(19) Europäisches Patentamt
European Patent Office
Office européen des brevets

(11) **EP 4 510 078 A1**

(12) **EUROPEAN PATENT APPLICATION**

(43) Date of publication:
**19.02.2025 Bulletin 2025/08**

(21) Application number: **24187209.2**

(22) Date of filing: **08.07.2024**

(51) International Patent Classification (IPC):
***G06T 11/00*** *(2006.01)*

(52) Cooperative Patent Classification (CPC):
**G06T 11/005; G06T 2211/452**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.08.2023 GB 202312466**

(71) Applicant: **Elekta Limited
Crawley
West Sussex RH10 9BL (GB)**

(72) Inventors:
• **MASON, Jonathan
Crawley, RH10 9RR (GB)**
• **STANCANELLO, Joseph
Crawley, RH10 9RR (GB)**
• **LONG, Andrew
Crawley, RH10 9RR (GB)**

(74) Representative: **Haseltine Lake Kempner LLP
One Portwall Square
Portwall Lane
Bristol BS1 6BH (GB)**

(54) **METHOD AND SYSTEM FOR IMAGE RECONSTRUCTION**

(57) A computer-implemented method of image reconstruction, the method comprising: acquiring projection data of a region of a patient, the projection data comprising projections representing measured ray intensities of attenuated rays of radiation emitted from a radiation source, passed through the patient, and detected at a detector, wherein the detector and the radiation source are rotated about an isocentre; performing opposing projection data correction to obtain modified projection data, the opposing projection data correction comprising modification of the ray intensities for projection data acquired at substantially 180 degrees offsets about the isocentre; and running an image reconstruction process on the modified projection data to obtain an image of the region of the patient.

| Acquire projection data of a region of a patient, the projection data comprising projections representing measured ray intensities of attenuated rays of radiation emitted from a radiation source, passed through the patient, and detected at a detector, wherein the detector and the radiation source are rotated about an isocentre. | 102 |
|---|---|
| Perform opposing projection data correction to obtain modified projection data, the opposing projection data correction comprising modification of the ray intensities for projection data acquired at substantially 180 degrees offsets about the isocentre. | 104 |
| Run an image reconstruction process on the modified projection data to obtain an image of the region of the patient. | 106 |

Figure 1

## Description

### Field of the Invention

The present invention relates to methods and systems for image reconstruction. More specifically, the present invention relates to a computer-implemented method for image reconstruction, using projection data relating to an object or a patient, and data processing apparatuses, computer programs, and non-transitory computer-readable storage mediums configured to execute methods of image reconstruction.

### Background of the Invention

Tomography is a non-invasive imaging technique, which allows visualisation of the internal structures of an object without the superposition of other structures that often afflict conventional projection images. For example, in a conventional chest radiograph, the ribs, heart, and lungs may be superimposed upon the same film, whereas a computed tomography (CT) slice is able to capture each body part in its three-dimensional position. Tomography has been applied across a range of disciplines, including medicine, physics, chemistry, and astronomy. While X-ray CT is perhaps the most familiar application of tomography, tomography may be performed using alternative imaging modalities, including ultrasound, magnetic resonance, nuclear-medicine, and microwave techniques.

Mathematically, the raw data acquired by a tomographic detector consists of multiple "projections" of the object being scanned. These projections are effectively the Radon transformation of the structure of the object. Reconstruction by means of tomographic reconstruction essentially involves solving the inverse Radon transformation.

Many practical reconstruction algorithms have been developed to implement the process of reconstruction of a three-dimensional object from its projections. These algorithms are designed largely based on the mathematics of the Radon transform, on statistical knowledge of the data acquisition process, and on geometry of the data computing means. Example reconstruction algorithms include filtered back projection algorithms and Fourier-domain reconstruction algorithms.

The presence of scatter in projection data acquired for tomographic reconstruction is currently a critical limiting factor in transmission CT modalities, such as Cone-Beam CT (CBCT) and fan-beam CT, as scatter induces significant degradation of the reconstructed image quality and accuracy.

In the field, such prominent methods have been introduced to address scatter correction, including: Monte Carlo techniques, which are accurate but slow; analytical techniques, which often lack the complexity to deal with heterogeneous objects (such as patients) accurately; and artificial intelligence (AI) scatter correction techniques, which are hampered with computationally intensive training requirements.

In the context of medical imaging and treatment, scatter represents a bottleneck problem in volumetric image guided therapy and adaptive radiation therapy. That is, although existing techniques yield promising results in scatter correction, these techniques are often limited to cases that require repeat scans and registration, and that rely on the assumption that the patient configuration, such as positioning and weight, has not changed during the course of treatment. In the context of medical imaging, high image quality, such as high contrast, homogeneity, and resolution, during radiation therapy is an important aid to accurately adjust, for instance, cancer treatments according to the current medical state of the patient. For adaptative radiation therapy, it is then desirable to utilise scanners that have large imaging field of views (FOVs). In this way, changes in patient configurations during the course of imaging and/or treatment may be captured.

The inventors have come to the realisation that there are imaging artefacts that afflict reconstructed image quality, which are particularly (but not exclusively) prevalent in large FOV (LFOV) reconstructed images. Further techniques are therefore required to correct for scatter-induced artefacts in reconstructed images.

### Summary of the Invention

The invention is defined in the independent claims, to which reference should now be made. Further features are set out in the dependent claims.

According to an aspect of the invention, there is provided a computer-implemented method of image reconstruction, so as to reduce image artefacts. The method comprises acquiring projection data of a region of a patient. The projection data may be acquired or received from external computational means, such as a data storage server, or directly from an imaging or treatment system. The projection data comprises projections, which are representative of measured ray intensities corresponding to attenuated rays of radiation. Beams of rays are emitted from some radiation source, passed through the region of the patient, and subsequently detected at a detector. The detector and the radiation source may be configured on or in a gantry, and may be rotated about an axis of the patient. Equivalently this rotation is about a radiation isocentre, which is the point (or centre of mass of a small bound region) in space through which rays intersect when the detector and the radiation source are rotated during beam-on. The techniques herein are equally applicable to static detectors and static radiation sources, arranged circumferentially about an axis of the patient. Similarly, the techniques herein are equally applicable to a combination of a static detector and rotating radiation sources, or a rotating detector and static radiation sources.

The method comprises performing opposing projection data correction. This correction process involves modification of the ray intensities for projection data for opposing rays, which are acquired at substantially 180 degrees offsets about the isocentre. That is, the method modifies ray intensities for projection data at a first position and the ray intensities for projection data at a second position, the second position being opposite (180 degree offset) the first position. Of course, the offset may not be exactly 180 degrees, due for example, to minor discrepancies in gantry configuration. Where, for example, the offset is not exactly 180 degrees, interpolation or extrapolation of data may be performed to ensure effective ray matching. The opposing projection data correction process - which may be referred to as opposing ray matching - results in modified projection data.

In this way, projection data may be modified to address or reduce the contribution of a scatter-induced image artefact. The artefact is thought to arise in reconstructed images due to scatter asymmetry. In an idealised scenario, where a ray passing from a first position to a second position is subject to the same scattering processes as a ray passing from the second position to the first position, the opposing projection data correction process would not be necessary. However, in realistic scenarios, scatter is asymmetric. This asymmetric scatter may be caused by asymmetric discrepancies in the imaging target, due to patient structure. This asymmetric scatter may be caused by the unavoidable contribution of a treatment couch, used to support a prone patient during imaging and treatment. And this asymmetric scatter may be caused by variations in source radiation intensities and detector efficacies at distinct positions around the gantry. LFOV images are particularly prone to the asymmetric scatter artefact due to the effective magnification of asymmetries when expanding the solid angle across the imaging region.

The method concludes with an image reconstruction process, performed on the modified projection data. Any conventional image reconstruction technique that used projection data may be used, so as to obtain a reconstructed image of the region of the patient.

The opposing projection data correction process is a simple method to match opposing rays, which routinely reduces the contribution of asymmetric scatter in reconstructed images. The opposing projection data correction process is a safe technique, in the sense that the technique only modifies projection data in the event that opposing rays differ. In an idealised scenario where the scatter for a path from a first point to a second is identical to that from the second point to the first, the technique may nonetheless be applied without fear of unnecessary data modification. The applicability of the correction process is therefore broad, and the technique may be simply integrated within existing image reconstruction methods and may be readily combined with other means of artefact correction.

Optionally, the projection data may be fan-beam computed tomography or cone-beam computed tomography, CBCT, data. These imaging modalities are commonly used in medical imaging. However, imaging modality should one in which the projection data may be acquired from opposing positions. Therefore, optionally, the projection data may be positron emission tomography, PET, data or single-photon emission computer tomography, SPECT, data.

Optionally, the method of image reconstruction may include further image enhancement or scatter reduction techniques. For instance, the image reconstruction method may perform glare deconvolution on the projection data, which involves deconvolution of a glare-spread function from projection data (in conjunction with a correction for so-called phantom-scatter) and which has been found useful for minimising artefacts. The reader is directed to the work of Poludniowski, G., et al (2011 Phys. Med. Biol. 56 1837) for further information. Preferably, other scatter reduction techniques are performed before opposing projection data correction, so as to ensure that projection matching is not lost or inadvertently misaligned.

Optionally, the measured ray intensities are a function of intensity signals acquired at detector elements of the detector. That is, the detector comprises a plurality of detector elements (e.g., individual pixels), and the measured ray intensities take into account the individual signals. In this way, one reduces the impact of random noise. For example, the measured ray intensities may be an average of underlying single detector element intensities.

Optionally, the image reconstruction method may be performed inline. That is, the method may correct projection data while the imaging system is acquiring projection data (rather than retroactive performance of the opposing projection data, performed on a complete projection data set). First ray intensities - acquired for a projection when the detector and the radiation source is positioned at a first orientation - are subject to opposing projection data correction following rotation (by substantially 180 degrees) of the detector and the radiation source to a second orientation and following acquisition of projection data at the second orientation. The projection data may be stored in a memory or buffer until required for opposing projection data correction. This enables scatter correction to be performed constantly, and enables rapid image reconstruction. Inline opposing projection data correction is particularly advantageous for adaptive radiotherapy.

Optionally, the method of image reconstruction may be applied in adaptive radiotherapy, which enables a patient's treatment to be changed, or adapted, to respond to a signal that additional information is known about the patient or that the patient has changed from the original state at the time of planning.

As set out above, the opposing projection data correction is not limited in application to any particular image reconstruction technique. Rather, any image reconstruction technique (or combination thereof) capable of reconstructing patient images from projection data is suitable. Optionally, the image reconstruction technique may involve a known Feldkamp, Davis, and Kress (FDK) reconstruction method. Optionally, the reconstruction technique may involve an iterative reconstruction method; such techniques - broadly speaking - begin with an assumed image, compute projections from the image, compare the original projection data, and update the image based upon the difference between the calculated and the actual projections. The advantages of the iterative approach include improved insensitivity to noise and the capability of reconstructing an optimal image in the case of incomplete data.

Optionally, the reconstruction technique may involve a Polyquant reconstruction, which may be regarded as a specific iterative reconstruction method. The reader is directed to the work of Mason, J. et al (arXiv:1706.08743, arXiv:1805.04411) for further information. Polyquant involves direct inference of some quantity of interest, whilst achieving beam hardening (nonlinearity due to the source having different energies) correction.

Optionally, the modification of ray intensities may include modification of first ray intensities for a projection at a first orientation (of the detector and the radiation source) using second ray intensities. The second ray intensities may be measured for a projection at a second orientation (of the detector and the radiation source), where the second orientation is substantially 180 degrees offset from the first orientation about the isocentre.

Optionally, the modification of ray intensities may include modification of first ray intensities for a projection at a first orientation using a first measured isocentral ray intensity and using a second measured isocentral ray intensity. The isocentral ray intensities correspond to (or are associated with) rays from amongst beams that each pass through the isocentre. The second ray (associated with the second isocentral ray intensity) is measured at a second orientation, substantially 180 degrees offset from the first orientation about the centre. The isocentral rays are not necessarily the rays within beams that are positioned in the geometric centre of the beam. The first and second isocentral rays are colinear. In effect, this technique uses the isocentral ray as a gauge to measure the mismatch between two opposing beams, and the correction is applied over the entire beam.

Optionally, where isocentral ray intensities are used in the modification of ray intensities, the modifications may include the perturbation as set out in formula 1 (that is, a perturbation of projection values to modified projection values).

$$CurrentProjection := CurrentProjection + \max(OpposingRay, Central\ Ray) - CentralRay \qquad (1)$$

Here, *CurrentProjection* are the first ray intensities for the projection at the first orientation. *CentralRay* is the first isocentral ray intensity. And *OpposingRay* is the second isocentral ray intensity.

Optionally, opposing projection data correction includes modification of the ray intensities and modification of scatter estimations for projection data. That is, the opposing projection data correction process may have regard to ray intensities and scatter estimations. In this sense, the projection data may include the measured intensities and, optionally, estimates of scatter or offset and gain images. For instance, rather than performing opposing ray matching in respect of ray intensities, the opposing projection data correction may perform opposing ray matching in respect of ray intensities and the scatter for each ray. This is particularly applicable where techniques such as Polyquant are used as in the subsequent image reconstruction process: a scatter estimate may not typically be "subtracted" from the measured projection, but instead "compensated" for during the iterative update of the reconstruction image. That is, opposing ray matching may match opposing ray intensities and opposing scatter estimates.

Optionally, the image reconstruction method is performed for multiple projections, acquired from different angles. That is, the method may include acquiring projection data and performing opposing projection data correction for a plurality of orientations of the detector and the radiation source. In this way, the asymmetric scatter-induced image artefact may be handled for all rotation angles, and eventual image quality may be improved.

Optionally, the image reconstruction method may account for flex within (or of) the gantry (and, specifically, the radiation source and the detector). The position of the detector for each gantry angle may vary due to flexing, and this flexing may be recorded through a calibration procedure. The opposing projection data correction process may involve determination of the position of each detector (or detector elements thereof) where each corresponding isocentral ray (from the corresponding radiation source) hits. By accounting for flex, the image reconstruction method may modify the projection data to account for lateral, vertical, and/or forward/backward detector offsets (e.g., from a calibrated, expected position) and/or may account for lateral, vertical, and/or forward/backward radiation source offsets. Similarly, the image reconstruction method may account for tilt (pitch, yaw, and/or roll) of the detector and/or the radiation source.

Embodiments of another aspect include a data processing apparatus comprising a memory storing computer-readable instructions and a processor. The processor (or controller circuitry) is configured to execute the instructions to carry out the computer-implemented method of image reconstruction.

Embodiments of another aspect include a computer program comprising instructions, which, when executed by computer, causes the compute to execute the computer-implemented method of image reconstruction.

Embodiments of another aspect include a non-transitory computer-readable storage medium comprising instructions, which, when executed by a computer, cause the compute to execute the computer-implemented method of image reconstruction.

The invention may be implemented in digital electronic circuitry, or in computer hardware, firmware, software, or in combinations thereof. The invention may be implemented as a computer program or a computer program product, i.e., a computer program tangibly embodied in a non-transitory information carrier, e.g., in a machine-readable storage device or in a propagated signal, for execution by, or to control the operation of, one or more hardware modules.

A computer program may be in the form of a stand-alone program, a computer program portion, or more than one computer program, and may be written in any form of programming language, including compiled or interpreted languages, and it may be deployed in any form, including as a stand-alone program or as a module, component, subroutine, or other unit suitable for use in a data processing environment.

The invention is described in terms of particular embodiments. Other embodiments are within the scope of the following claims. For example, the steps of the invention may be performed in a different order and still achieve desirable results.

Elements of the invention have been described using the terms "processor", "input device" etc. The skilled person will appreciate that such functional terms and their equivalents may refer to parts of the system that are spatially separate but combine to serve the function defined. Equally, the same physical parts of the system may provide two or more of the functions defined. For example, separately defined means may be implemented using the same memory and/or processor as appropriate.

**Brief Description of the Drawings**

Reference is made, by way of example only, to the accompanying drawings in which:

Figure 1 is a flow chart of a method of image reconstruction, according to an embodiment;
Figure 2 is a diagram of the geometric arrangement for fan beam projection or cone beam projection scanning and reconstruction;
Figure 3 is a diagram of a cone beam CT scanner;
Figure 4 is a diagram of the geometric arrangement for opposing projection data correction, applied to fan beam or cone beam CT projection data;
Figure 5 is a collection of reconstructed images, reconstructed with and without opposing projection data correction on a torso phantom;
Figure 6 is a collection of reconstructed images, reconstructed with and without opposing projection data correction on first clinical data;
Figure 7 is a collection of reconstructed images, reconstructed with and without opposing projection data correction on second, third, and fourth clinical data;
Figure 8 is a radiotherapy system, suitable for implementing image reconstruction according to embodiments; and
Figure 9 is a radiotherapy device or apparatus, suitable for implementing image reconstruction according to embodiments.

**Detailed Description**

Treatment planning typically involves performing an optimization procedure to optimize a number of parameters, the purpose being to provide a sufficiently high dose to the target region (or planned target volume, PTV) while reducing the dose to the surrounding healthy tissue. By balancing the different treatment-planning objectives, for example by prioritising treatment-planning objectives that relate to the dose coverage at the target region and then treatment planning that relate to sparing surrounding regions, a clinically suitable plan may be obtained. However, a user may ask for unrealistically low defined dose values in surrounding regions, which negatively impacts the optimization procedures and - in turn - negatively effects the treatment planning process.

As an example, a CT image may be reconstructed from multiple projections that are acquired as an X-ray source rotates around the object (a patient). The acquisition geometry may be defined by the acquisition FOV, which may be determined by the beam angle, and may determine the maximum possible size of reconstructed image. For head CT scans, a FOV may be on the order of 250 mm. A typical LFOV, which is commonly used for whole-body scanning for example, may be of a diameter on the order of 500 mm. More specifically, a system for which the techniques herein are particularly applicable has a FOV diameter of around 510 to 520 mm. A medium FOV (MFOV) system may have a FOV of a diameter on the order of 400 mm. More specifically, a system for which the techniques herein are particularly applicable has a FOV diameter of around 410 to 420 mm. The inventors have come to the realisation that users often avoid use of LFOV and MFOV configurations when scanning, due to an increase in the prevalence and effect of image artefacts.

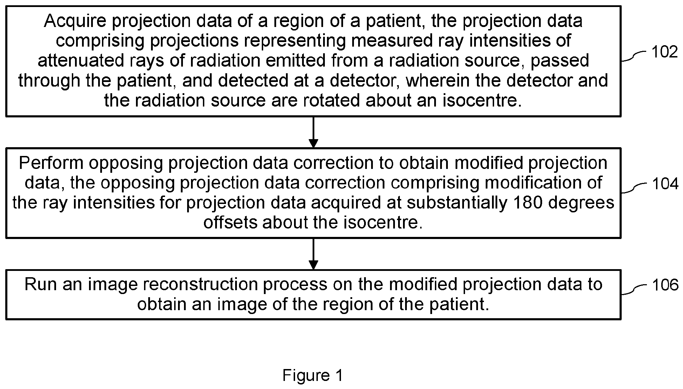

FIG. 1 shows a general method for image reconstruction, which includes opposing ray matching and which addresses asymmetric scatter induced image artefacts particularly prevalent in LFOV systems and medium FOV (MFOV) systems.

At step 102, a computer acquires projection data of a patient. The projection data comprises projections representing measured ray intensities of attenuated rays of radiation. The rays are emitted from a radiation source, passed through the patient, and detected at a detector. The detector and the radiation source are rotated about an isocentre of the system.

At step 104, the computer performs opposing projection data correction (or opposing ray matching) to obtain modified projection data. The opposing projection data correction process comprises modification of the ray intensities for projection data acquired at substantially 180 degrees offsets about the isocentre. The opposing projection data process may be considered as a data pre-processing step, to be performed on projection data prior to image reconstruction.

At step 106, the computer runs an image reconstruction process of the modified projection data. In turn, the computer obtains an image of the region of the patient. Where asymmetric scatter is present in the raw projection data, the opposing projection data correction process ensures that the associated artefact is removed or at least significantly reduced in the reconstructed image.

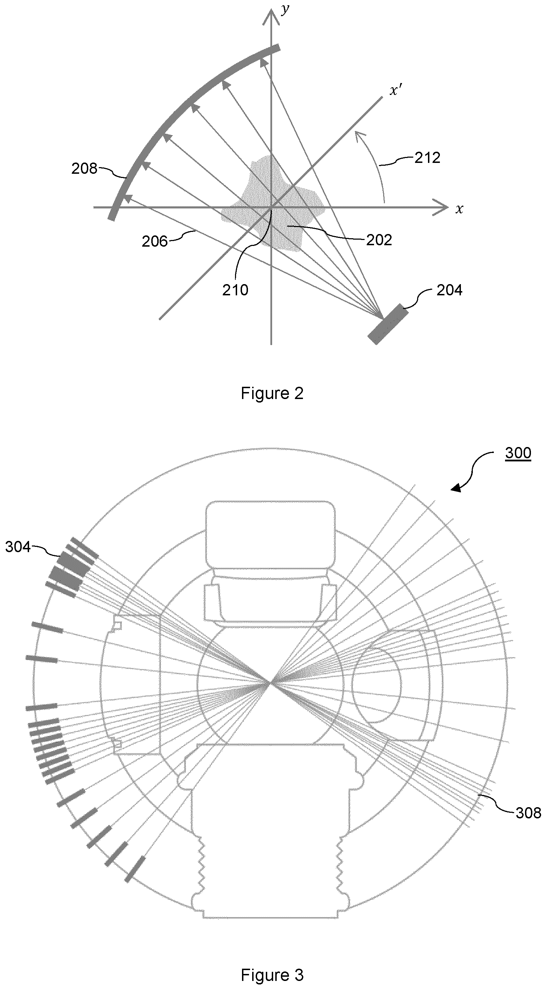

FIG. 2 illustrates the geometry of a typical fan beam or cone beam projection acquisition setup, as may be used in image reconstruction techniques according to embodiments. For simplicity, only the x-y plane is illustrated; the skilled reader will appreciate that - particularly for CBCT imaging - the beam of rays extends also into the z-axis, orthogonal to the x-y plane. In this example, the acquisition setup captures projection data of object 202.

X-ray source 204 generates and emits X-rays 206 towards object 202. In fan beam CT and CBCT, X-rays may be considered as a beam of rays, emitted from a point source. Other imaging modalities (such as parallel beam systems) may include multiple sources or extended sources, from which X-rays may be emitted. Detector (or detectors) 208 capture projections, which are sets of line integrals along paths that radiate from the source 204.

Multiple projections of the image may be acquired from different angles by rotating the source 204 and detector 208 around the centre of the image 210. In the present example, the source 204 and detector 208 may be rotated arcuately along orbital path 212. In this way, the x-y plane may be rotated counter clockwise around the point of origin (or centre of the image 210) in a manner that keeps the mutual positional relationship between source 204 and detector 208 when passing through the orbital path 212. Other configurations of imaging systems are, of course, feasible; for instance, the source may be configured to rotate and a complete ring of detectors may be configured to capture projections, or there may be multiple sources arranged circumferentially around a complete ring of detectors. Further, the imaging system may rotate the source along a helical path, so as to capture projection data along the axis of the object 202 of interest (along the z-axis in the present example).

The attenuation of the intensity of the rays that pass through the object 202 may be measured by processing signals received from the detector 208. By making projective measurements at a series of different projection angles through the object 202, a sinogram may be constructed from the projection data, mapping the spatial dimension of the detector array to the projection angle dimension. The intensity attenuation resulting from a particular volume with the object will trace out a sine wave for the spatial dimension along the detector perpendicular to the rotation axis of the system. Volumes of the object farther from the centre of rotation correspond to sine waves with greater amplitudes than those corresponding to volumes nearer the centre of rotation. The phase of each sine wave in the sinogram corresponds to the relative angular positions with respect to the rotation axis. By performing an image reconstruction technique (such as an inverse Radon transform) on the projection data in the sinogram, one may reconstruct an image, where the reconstructed image corresponds to a cross-sectional slice of the object 202.

The detector 208 may comprise underlying detector elements (such as pixels or bins of pixels), rather than a single active region. The measured ray intensities may then be taken a function of underlying intensity signals acquired at detector elements of the detector. This is advantageous in that one reduces the impact of random noise, which may plague an intensity (particular a low intensity) measured at a single detector element. For example, the measured ray intensities may be an average (mean, mode or median) of underlying single detector element intensities.

FIG. 3 illustrates the geometry of a fan beam or cone beam projection acquisition setup, as incorporated into a medical CT scanner 300. Radiation sources 304 emit beams of X-ray radiation, which may pass through the patient supported on a couch within the CT scanner (not depicted). Detectors 308 capture attenuated X-rays. The radiation sources 304 and detectors 308 may be configured to rotate within a gantry of the CT scanner, so as to acquire projective measurements at a series of different projection angles. As shown, the angular separation between measurement orientations need not be equal.

The image reconstruction method may account for flex within (or of) the gantry. For instance, in one implementation, the flex may be considered when determining where the isocentral ray hits the detector. In turn, the method may determine the intensity at the isocentral location through interpolation. As an example, assume the centre of detector at gantry angle t has coordinates (0,0); for 2D detector translation flex, the image reconstruction method may perform the following process.

1. Isocentral ray hits detector at (-dx, -dy), where dx and dy are per-projection offsets of the detector found through calibration.

    2. Linearly interpolate sub-pixel projection value at (-dx, -dy).

    3. Store subpixel value and use in opposing projection data correction.

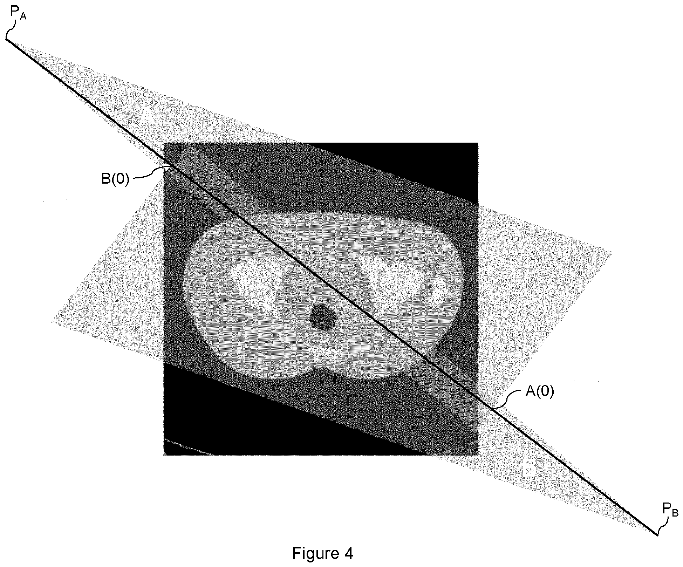

FIG. 4 is a diagram of geometric considerations for opposing projection data correction, applied to fan beam or cone beam CT projection data. The object of attention in the present example is a patient torso. For example, the patient may be prone on a couch of a CT scanner, such as that depicted in Fig. 3. An X-ray source, when positioned at first position, $P_A$, generates a beam of rays, which passes through the patient torso and is detected at detectors on the opposite side of the patient (not depicted). The detected projections, $A$, are signal intensities for each ray within the beam. As shown, the beam in this example is a fan or cone beam, which diverges as it passes through the patient.

The radiation source and the detector are then rotated by 180 degrees (or the nearest approximation thereof) to the opposing side of the patient, such that the source is at a second position, $P_B$. The source similarly generates a beam of rays, which passes through the patient torso and is detected at detectors on the opposing side of the patient. The detected projections, $B$, are signal intensities for each ray within the beam. There exists colinear isocentral rays within each beam (with projections $A(0)$ and $B(0)$), which pass through the isocentre of the system. The opposing projection data correction process modifies the beam projections pairwise, such that the ray intensities, $A$, of the first beam are modified in respect of the ray intensities, $B$, of the second beam, and vice versa. For example, the ray intensities $A$ and $B$ may be modified or perturbed according to formulae 2 and 3:

$$A := A + \max\big(A(0), B(0)\big) - A(0) \qquad (2)$$

$$B := B + \max\big(A(0), B(0)\big) - B(0) \qquad (3)$$

In other words, if $A(0) > B(0)$, then $A$ is unchanged and ray intensities $B$ are modified with a bias term equivalent to the difference in the isocentral ray intensities, $A(0)$ and $B(0)$. Similarly, if $B(0) > A(0)$, then $B$ is unchanged and ray intensities $A$ are modified with the bias term. More generally, the formulae may be expressed as formula (1), set about above.

The purpose of the ray modifications according to formulae (2) and (3) is to account for differences between the projections at opposing ray orientations. This difference gives rise artefacts in reconstructed images, caused by scatter asymmetry. In an idealised configuration, the isocentral rays (passing through the isocentre of the system) should match. The opposing projection data correction technique herein provides effective uniform scatter correction.

In the present example, a single opposing pair of positions (of radiation source and detector) is considered. The skilled reader will appreciate that the opposing projection data correction technique may be applied to all data for which projection data is acquired at opposite sides of the gantry (i.e., wherever the detector and radiation source are positioned at a substantially 180 degree offset).

The specific form of the opposing projection data correction algorithm is not limited to formulae (2) and (3) above. Another example manner in which ray intensities $A$ and $B$ may be modified or perturbed as set out in formulae (4) and (5):

$$A := A + \frac{B(0) - A(0)}{2} \qquad (4)$$

$$B := B + \frac{A(0) - B(0)}{2} \qquad (5)$$

Following opposing projection data correction, the modified projection data is used to perform image reconstruction and thereby acquire an image of the target object (a patient torso in the present example). In the following examples, the inventors have applied the Feldkamp, Davis, and Kress, FDK, analytical algorithm for the reconstruction of CT images. The FDK algorithm is, in essence, a filtered back-projection, which applies a filter in the frequency domain before back-projecting the projection data. The reader is directed to the medical literature, where further details of the FDK algorithm are readily available. Equally, the opposing projection data correction process may be readily integrated into other forms of image reconstruction, such as direct Fourier reconstruction techniques and iterative reconstruction techniques, such as statistical reconstruction and algebraic reconstruction.

The opposing projection data correction may be supplemented with further artefact reduction techniques. For instance, the inventors have found that glare correction via glare deconvolution on the projection in particularly advantageous. Glare deconvolution involves deconvolution of a glare-spread function from projection data (potentially in conjunction with a correction for so-called phantom-scatter) and which has been found useful for minimising artefacts. The reader is directed to the work of Poludniowski, G., et al (2011 Phys. Med. Biol. 56 1837) for further information.

The inventors have found that scatter correction is another particularly advantageous supplementary artefact reduction technique. Scatter correction involves the estimation and compensation for scattered X-rays (photons) that undergo interaction but still reach the detector, and in turn corrupt the measurement. Scatter estimation approaches can be broadly characterised as "projection based" or "image based".

In projection based scatter estimation, the scatter component may be directly inferred in the measurement domain, such as a uniform scatter estimation behind the patient (see, for example, the work of Boellaard, R., et al., DOI: 10.1016/S0167-8140(97)00073-X), a convolutional kernel (see, for example, the work of Ohnesorge, B., et al. DOI: 10.1007/s003300050710), or a deep learning approach (see, for example, the work of Maier, J., et al. DOI: 10.1007/s10921-018-0507-z).

In image based scatter estimation, a physical simulation may be used to compute the expected scatter with, for example, Monte Carlo code (see, for example, the work of Xu, Y. et al. DOI: 10.1088/0031-9155/60/9/3567).

In some cases, the scatter estimate may be subtracted from the measurements, and in other cases the scatter estimate may be considered in a statistical model during a model-based iterative reconstruction (see, for example, the work of Erdogan, H. & Fessler, J. A., DOI: 10.1109/42.802758).

Preferably, supplementary artefact reduction techniques (such as scatter correction) are applied before opposing projection data correction, such that any supplementary artefact reduction techniques do not alter the projections in a manner than may cause misalignment of rays that are substantially 180 degrees offset from one another.

In one implementation, the inventors have utilised the programming language and computing environment, MATLAB to realise the above method of image reconstruction, including opposing projection data correction. In a second implementation, the inventors have utilised the programming language C++, and in a third implementation, the inventors have utilised C++ in combination with the parallel computing platform and API, CUDA. Opposing projection data correction may then be performed using parallel processing. In implementations, the projection data are stored in GPU memory, and are asynchronously updated as required.

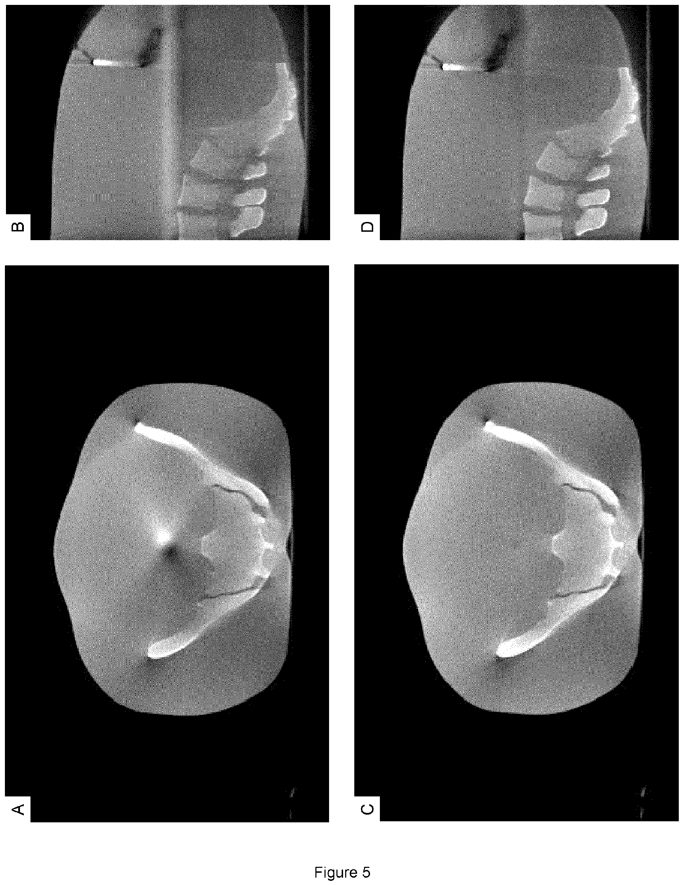

FIG. 5 shows the effect of the opposing projection data process on example projection data. In this example, the object of interest is a torso phantom. Images are reconstructed from projection data using the FDK algorithm. Panel A illustrates a reconstructed image slice, for projection data acquired in a transverse plane of the phantom, without use of the opposing projection data correction process. The asymmetric scatter artefact (induced by scatter asymmetry in opposing ray projections) is clearly visible in the centre of the image as a bright spot and a dark spot in close proximity. Panel B illustrates the torso phantom in the sagittal plane of the phantom, without use of the opposing projection data correction process. The asymmetric scatter artefact takes the form of rod-like artefacts spanning the long axis of the phantom (due to the image being reconstructed from transverse slices, each with the asymmetric scatter artefact).

Panel C illustrates a reconstructed image slice, for projection data acquired in a transverse plane of the phantom. Here, reconstruction process uses projection data modified with the opposing projection data correction process. Panel D illustrates the torso phantom in the sagittal plane of the phantom, using the opposing projection data correction process. Evidently, the opposing projection data correction process significantly reduces the impact of asymmetric scatter in projection data, with minimal effect on the remainder of the image.

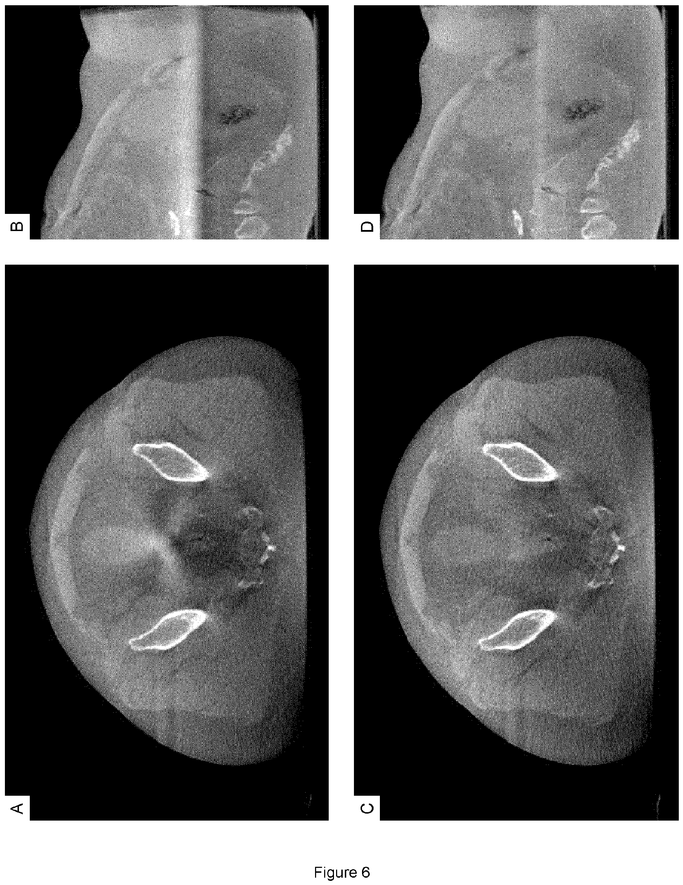

FIG. 6 shows the effect of the opposing projection data process on example projection data for a clinical patient. Again, images are reconstructed from projection data using the FDK algorithm, Panel A illustrates a reconstructed transverse image of the patient torso, and panel B illustrates a reconstructed sagittal slice of the patient torso, each without use of the opposing projection data correction process. While not as pronounced as the previous phantom example, the asymmetric scatter artefact is present as a bright spot and a bright rod in the transverse and sagittal slices, respectively.

Panel C illustrates a reconstructed transverse image of the patient torso, and panel D illustrates a reconstructed sagittal slice of the patient torso, each with use of the opposing projection data correction process. As with the previous phantom example, the opposing projection data correction process significantly reduces the impact of asymmetric scatter in projection data. Note that in the transverse reconstructed slices, there exists band-like artefacts that span the horizontal direction; these are likely under-scan artefacts, which occur when the angular arc spanned by the detector and the radiation source is less than 360 degrees.

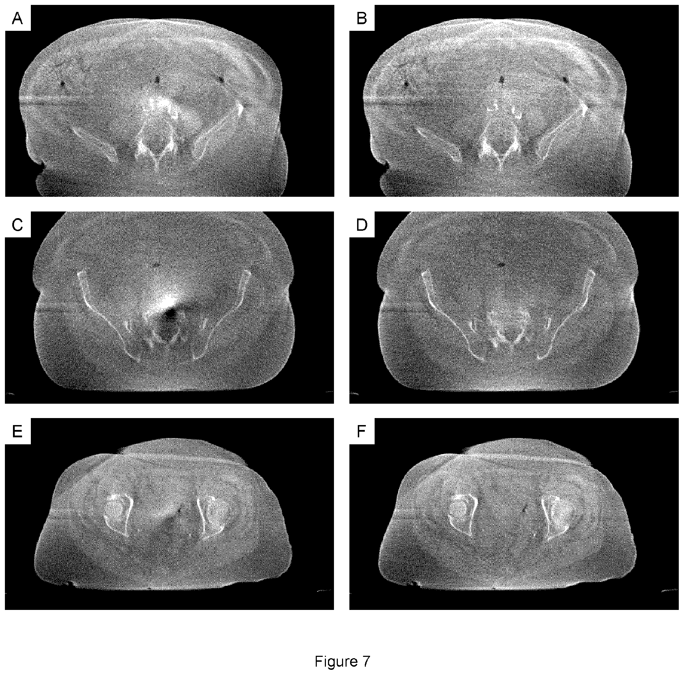

FIG. 7 shows the effect of the opposing projection data process for further example clinical projection data. Panels A, C and E show reconstructed transverse images without use of the opposing projection data correction process for three distinct patients. Panels B, D and F show reconstructed transverse images for the same three patients, where the opposing projection data correction process is applied. Again, in each case, the opposing projection data correction process significantly reduces the impact of asymmetric scatter in projection data.

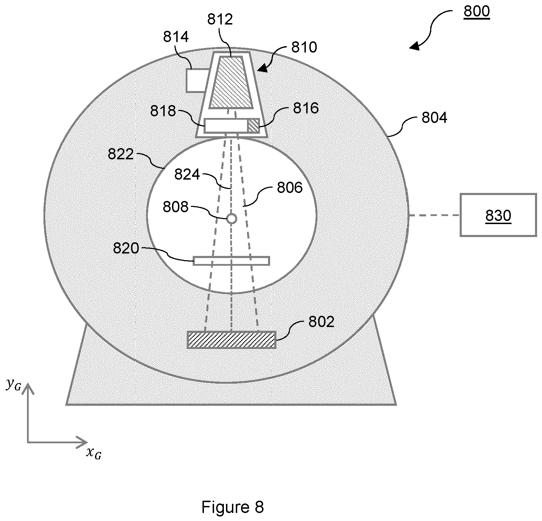

FIG. 8 depicts a radiotherapy apparatus, suitable for acquiring projection data for image reconstruction according to embodiments. The cross-section through radiotherapy apparatus 800 includes a radiation head 810 and a beam receiving apparatus (detector) 802, both of which are attached to a gantry 804. The radiation head 810 includes a radiation source 812, which emits a beam of radiation 806. The radiation head 810 also includes a beam shaping apparatus 818, which controls the size and shape of the radiation field associated with the beam.

The beam receiving apparatus 802 is configured to receive radiation emitted from the radiation head 810, for the purpose of absorbing and/or measuring the beam of radiation. In the view shown, the radiation head 810 and the beam receiving apparatus 802 are positioned diametrically opposed to one another.

The gantry 804 is rotatable, and supports the radiation head 810 and the beam receiving apparatus 802 such that they are rotatable around an axis of rotation 808, which may coincide with the patient longitudinal axis. The gantry provides rotation of the radiation head 810 and the beam receiving apparatus 802 in a plane perpendicular to the patient longitudinal axis (e.g., a sagittal plane). Three gantry directions $x_G$, $y_G$, $z_G$ may be defined such that the $y_G$ direction is perpendicular with the gantry axis of rotation. The $y_G$ direction extends from a point on the gantry corresponding to the radiation head 810, towards the axis of rotation of the gantry. Therefore, from the patient frame of reference, the $y_G$ direction rotates around as the gantry rotates.

The radiotherapy apparatus 800 also includes a support surface or couch 820 on which a subject (or patient) is supported during radiotherapy treatment or image acquisition. The radiation head 810 is configured to rotate around the axis of rotation 808 such that the radiation head 810 directs radiation towards the subject from various angles around the subject in order to spread out the radiation dose received by healthy tissue to a larger region of healthy tissue while building up a prescribed dose of radiation at a target region.

The radiotherapy apparatus 800 is configured to deliver a radiation beam towards a radiation isocentre, which is substantially located on the axis of rotation 808 at the centre of the gantry 804 regardless of the angle at which the radiation head 810 is placed.

The rotatable gantry 804 and radiation head 810 are dimensioned so as to allow a central bore 822 to exist. The central bore 822 provides an opening, sufficient to allow a subject to be positioned therethrough without the possibility of being incidentally contacted by the radiation head 810 or other mechanical components as the gantry rotates the radiation head 810 about the subject.

The radiation head 810 emits the radiation beam 806 along a beam axis 824 (or radiation axis or beam path), where the beam axis 824 is used to define the direction in which the radiation is emitted by the radiation head 810. The radiation beam 806 is incident on the beam receiving apparatus 802, which may include at least one of a beam stopper and a radiation detector. The beam receiving apparatus 802 is attached to the gantry 804 on a diametrically opposite side to the radiation head 810 to attenuate and/or detect a beam of radiation after the beam has passed through the subject.

The radiation beam axis 824 may be defined as, for example, a centre of the radiation beam 806 or a point of maximum intensity.

The beam shaping apparatus 818 delimits the spread of the radiation beam 806. The beam shaping apparatus 818 is configured to adjust the shape and/or size of a field of radiation produced by the radiation source. The beam shaping apparatus 818 does this by defining an aperture (also referred to as a window or an opening) of variable shape to collimate the radiation beam 806 to a chosen cross-sectional shape. In this example, the beam shaping apparatus 818 may be provided by a combination of a diaphragm and an MLC. Beam shaping apparatus 818 may also be referred to as a beam modifier.

The radiotherapy apparatus 800 may be configured to deliver both coplanar and non-coplanar (also referred to as tilted) modes of radiotherapy treatment. In coplanar treatment, radiation is emitted in a plane that is perpendicular to the axis of rotation of the radiation head 810. In non-coplanar treatment, radiation is emitted at an angle that is not perpendicular to the axis of rotation. In order to deliver coplanar and non-coplanar treatment, the radiation head 810 may move between at least two positions, one in which the radiation is emitted in a plane which is perpendicular to the axis of rotation (coplanar configuration) and one in which radiation is emitted in a plane which is not perpendicular to the axis of rotation (non-coplanar configuration).

In the coplanar configuration, the radiation head 810 is positioned to rotate about a rotation axis and in a first plane. In the non-coplanar configuration, the radiation head is tilted with respect to the first plane such that a field of radiation produced by the radiation head is directed at an oblique angle relative to the first plane and the rotation axis. In the non-coplanar configuration, the radiation head 810 is positioned to rotate in a respective second plane parallel to and displaced from the first plane. The radiation beam is emitted at an oblique angle with respect to the second plane, and therefore as the radiation head rotates the beam sweeps out a cone shape.

In one configuration, the beam receiving apparatus 802 may remain in the same place relative to the rotatable gantry when the radiotherapy apparatus is in both the coplanar and non-coplanar modes. Therefore, the beam receiving apparatus 802 is configured to rotate about the rotation axis in the same plane in both coplanar and non-coplanar modes. This may be the same plane as the plane in which the radiation head rotates. In alternative configurations, the beam receiving apparatus 801 may also rotate.

The beam shaping apparatus 810 is configured to reduce the spread of the field of radiation in the non-coplanar configuration in comparison to the coplanar configuration.

The radiotherapy apparatus 800 includes a controller 830, which is programmed to control the radiation source 812, beam receiving apparatus 806 and the gantry 802. Controller 830 may perform functions or operations such as treatment planning, treatment execution, image acquisition, image processing, motion tracking, motion management, and/or other tasks involved in a radiotherapy process.

Controller 830 is programmed to control various components of apparatus 800, such as gantry 804, radiation head 810, beam receiving apparatus 802, and support surface 820, so as to acquire projection data suitable for image reconstruction.

Hardware components of controller 830 may include one or more computers (e.g., general purpose computers, workstations, servers, terminals, portable/mobile devices, etc.); processors (e.g., central processing units (CPUs), graphics processing units (GPUs), microprocessors, digital signal processors (DSPs), field programmable gate arrays (FPGAs), special-purpose or specially-designed processors, etc.); memory/storage devices such as a memory (e.g., read-only memories (ROMs), random access memories (RAMs), flash memories, hard drives, optical disks, solid-state drives (SSDs), etc.); input devices (e.g., keyboards, mice, touch screens, mics, buttons, knobs, trackballs, levers, handles, joysticks, etc.); output devices (e.g., displays, printers, speakers, vibration devices, etc.); circuitries; printed circuit boards (PCBs); or other suitable hardware. Software components of controller 830 may include operation device software, application software, etc.

The radiation head 810 may be connected to a head actuator 814, which is configured to actuate the radiation head 810, for example between a coplanar configuration and one or more non-coplanar configurations, or for example to actuate the radiation source 812 and/or detector 802 in response to detection of flex. This may involve translation and rotation of the radiation head 810 relative to the gantry. In some implementations, the head actuator may include a curved rail along which the radiation head 810 may be moved to adjust the position and angle of the radiation head 810. The controller 830 may control the configuration of the radiation head 830 via the head actuator 814.

The beam shaping apparatus 818 includes a shaping actuator 816. The shaping actuator is configured to control the position of one or more elements in the beam shaping apparatus 818 in order to shape the radiation beam 806. In some implementations, the beam shaping apparatus 816 includes an MLC, and the shaping actuator 816 includes means for actuating leaves of the MLC. The beam shaping apparatus 818 may further comprise a diaphragm, and the shaping actuator 816 may include means for actuating blocks of the diaphragm. The controller 830 may control the beam shaping apparatus 818 via the shaping actuator 816.

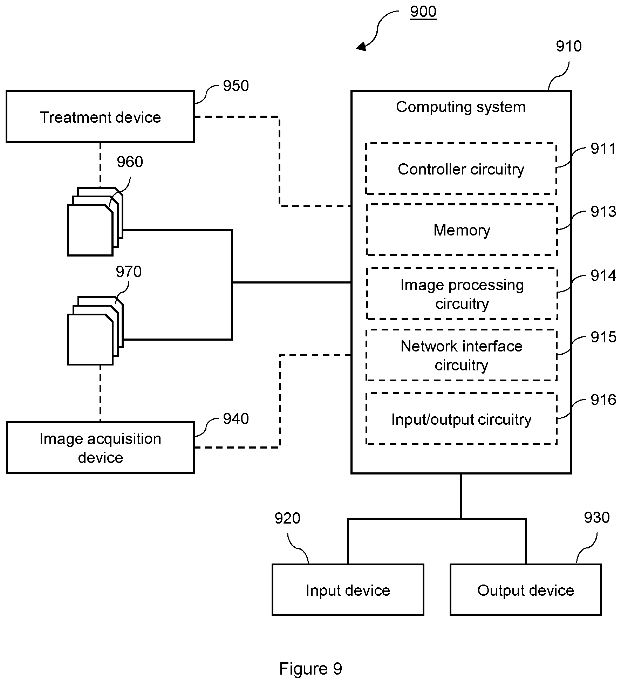

FIG. 9 is a block diagram of an implementation of a radiotherapy system 900, suitable for executing methods for image reconstruction according to embodiments. The example radiotherapy system 900 comprises a computing system 910 within which a set of instructions, for causing the computing system 910 to perform the method (or steps thereof) discussed herein, may be executed. The computing system 910 may implement an image reconstruction system. The computing system 910 may also be referred to as a computer. In particular, the methods described herein may be implemented by a processor or controller circuitry 911 of the treatment planning system 910.

The computing system 910 shall be taken to include any number or collection of machines, e.g., computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 910 includes controller circuitry 911 and a memory 913 (e.g., read-only memory (ROM), flash memory, dynamic random access memory (DRAM) such as synchronous DRAM (SDRAM) or Rambus DRAM (RDRAM), etc.). The memory 913 may comprise a static memory (e.g., flash memory, static random access memory (SRAM), etc.), and/or a secondary memory (e.g., a data storage device), which communicate with each other via a bus (not shown). Memory 913 may be used to store or buffer projection data until required for opposing projection data correction (e.g., until radiation source and detector have been rotated by substantially 180 degrees and opposing ray data has been acquired).

Controller circuitry 911 represents one or more general-purpose processors such as a microprocessor, central processing unit, accelerated processing units, or the like. More particularly, the controller circuitry 911 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 911 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 911 is configured to execute the processing logic for performing the operations and steps discussed herein.

The computing system 910 may further include a network interface circuitry 915. The computing system 910 may be communicatively coupled to an input device 920 and/or an output device 930, via input/output circuitry 916. In some implementations, the input device 920 and/or the output device 930 may be elements of the computing system 910. The input device 920 may include an alphanumeric input device (e.g., a keyboard or touchscreen), a cursor control device (e.g., a mouse or touchscreen), an audio device such as a microphone, and/or a haptic input device. The output device 930 may include an audio device such as a speaker, a video display unit (e.g., a liquid crystal display (LCD) or a cathode ray tube (CRT)), and/or a haptic output device. In some implementations, the input device 920 and the output device 930 may be provided as a single device, or as separate devices.

In some implementations, the computing system 910 may comprise image processing circuitry 914. Image processing circuitry 914 may be configured to process image data 970 (e.g., images, imaging data, projections, projection data), such as medical images obtained from one or more imaging data sources, a treatment device 950 and/or an image acquisition device 940. Image processing circuitry 914 may be configured to process, or pre-process, image data 970. For example, image processing circuitry 914 may convert received image data into a particular format, size, resolution or the like. Image processing circuitry 914 may be configured to perform image reconstruction. In some implementations, image processing circuitry 914 may be combined with controller circuitry 911.

In some implementations, the radiotherapy system 900 may further comprise an image acquisition device 940 and/or a treatment device 950. The image acquisition device 940 and the treatment device 950 may be provided as a single device. In some implementations, treatment device 950 is configured to perform imaging, for example in addition to providing treatment and/or during treatment.

Image acquisition device 940 may be configured to perform positron emission tomography (PET), computed tomography (CT), magnetic resonance imaging (MRI), single positron emission computed tomography (SPECT), X-ray, and the like.

Image acquisition device 940 may be configured to output image data 970, which may be accessed by computing system 910. Treatment device 950 may be configured to output treatment data 960, which may be accessed by computing system 910. Treatment data 960 may be obtained from an internal data source (e.g., from memory 913) or from an external data source, such as treatment device 950 or an external database.

The various methods described above may be implemented by a computer program. The computer program may include computer code (e.g., instructions) arranged to instruct a computer to perform the functions of one or more of the various methods described above. For example, the steps of the methods described in relation to Fig. 1 may be performed by the computer code. The steps of the methods described above may be performed in any suitable order. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions may also reside, completely or at least partially, within the memory 913 and/or within the controller circuitry 911 during execution thereof by the computing system 910, the memory 913 and the controller circuitry 911 also constituting computer-readable storage media.

In an implementation, the modules, components and other features described herein may be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components may be implemented as firmware or functional circuitry within hardware devices. Further, the modules and components may be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining", "identifying", "obtaining", "accessing", or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

While certain embodiments have been described, these embodiments have been presented by way of example only, and are not intended to limit the scope of the inventions. Indeed, the novel methods and apparatuses described herein may be embodied in a variety of other forms; furthermore, various omissions, substitutions and changes in the form of methods and apparatus described herein may be made.

**Claims**

1. A computer-implemented method of image reconstruction, the method comprising:

   acquiring projection data of a region of a patient, the projection data comprising projections representing measured ray intensities of attenuated rays of radiation emitted from a radiation source, passed through the patient, and detected at a detector, wherein the detector and the radiation source are rotated about an isocentre;
   performing opposing projection data correction to obtain modified projection data, the opposing projection data correction comprising modification of the ray intensities for projection data acquired at substantially 180 degrees offsets about the isocentre; and
   running an image reconstruction process on the modified projection data to obtain an image of the region of the patient.

2. The method of claim 1, wherein the projection data is fan-beam or cone-beam computed tomography, CBCT, data.

3. The method of claim 1, wherein the projection data is positron emission tomography, PET, data or single-photon emission computer tomography, SPECT, data.

4. The method of any preceding claim, further comprising performing glare deconvolution on the projection data and/or scatter correction.

5. The method of any preceding claim, wherein the measured ray intensities are a function of intensity signals acquired at detector elements of the detector.

6. The method of any preceding claim, wherein acquiring projection data and performing opposing projection data correction are performed inline, such that first ray intensities for a projection at a first orientation of the detector and the radiation source are subject to the opposing projection data correction following rotation of the detector and radiation source by substantially 180 degrees about the isocentre to a second orientation and acquisition of projection data at the second orientation.

7. The method of any preceding claim, for use in adaptive radiotherapy.

8. The method of any preceding claim, wherein the reconstruction technique is any of: a Feldkamp Davis and Kress, FDK, reconstruction technique; an iterative reconstruction technique; and a Polyquant reconstruction technique.

9. The method of any preceding claim, wherein the modification of ray intensities comprises modification of first ray intensities for a projection at a first orientation of the detector and the radiation source using second ray intensities, the second ray intensities being measured for a projection at a second orientation of the detector and the radiation source, the second orientation being substantially 180 degrees offset from the first orientation about the isocentre.

10. The method of any preceding claim, wherein the modification of ray intensities comprises modification of first ray intensities for a projection at a first orientation of the detector and the radiation source using a first isocentral ray intensity corresponding to a first ray that passes through the isocentre and using a second isocentral ray intensity corresponding to a second ray that passes through the isocentre, the second ray being measured at a second orientation, the second orientation being substantially 180 degrees offset from the first orientation about the isocentre, optionally wherein the modifications comprise application of the following perturbation:

$$CurrentProjection \coloneqq CurrentProjection + \max(OpposingRay, Central\ Ray) - CentralRay,$$

where *CurrentProjection* are the first ray intensities for the projection at the first orientation, *CentralRay* is the first isocentral ray intensity, and *OpposingRay* is the second isocentral ray intensity.

11. The method of any preceding claim, wherein opposing projection data correction comprises modification of the ray intensities and modification of scatter estimations for projection data acquired at substantially 180 degrees offsets about the isocentre.

12. The method of any preceding claim, wherein acquiring projection data and performing opposing projection data correction are performed for a plurality of orientations of the detector and the radiation source.

13. The method of any preceding claim, wherein opposing projection data correction comprises adjustment for any flex of the radiation source and/or the detector.

14. A data processing apparatus comprising a memory storing computer-executable instructions, and a processor configured to execute the instructions to carry out the method of any of claims 1 to 13.

15. A computer-readable medium comprising instructions which, when executed by a computer, cause the computer to carry out the method of any of claims 1 to 13.

| Acquire projection data of a region of a patient, the projection data comprising projections representing measured ray intensities of attenuated rays of radiation emitted from a radiation source, passed through the patient, and detected at a detector, wherein the detector and the radiation source are rotated about an isocentre. | 102 |

↓

| Perform opposing projection data correction to obtain modified projection data, the opposing projection data correction comprising modification of the ray intensities for projection data acquired at substantially 180 degrees offsets about the isocentre. | 104 |

↓

| Run an image reconstruction process on the modified projection data to obtain an image of the region of the patient. | 106 |

Figure 1

Figure 2

Figure 3

Figure 4

Figure 5

Figure 6

Figure 7

EP 4 510 078 A1

Figure 8

20

Figure 9

Europäisches
Patentamt

European
Patent Office

Office européen
des brevets

**EUROPEAN SEARCH REPORT**

Application Number

EP 24 18 7209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/177713 A1 (KOHLER THOMAS [DE] ET AL) 2 August 2007 (2007-08-02) * the whole document * | 1-15 | INV. G06T11/00 |
| X | US 2012/207370 A1 (FAHIMIAN BENJAMIN POOYA [US] ET AL) 16 August 2012 (2012-08-16) * the whole document * | 1-15 | |
| X | WO 97/33516 A1 (ANALOGIC CORP [US]) 18 September 1997 (1997-09-18) * the whole document * | 1-15 | |

|  |
|---|
| TECHNICAL FIELDS SEARCHED (IPC) |
| G06T |

The present search report has been drawn up for all claims

| Place of search | Date of completion of the search | Examiner |
|---|---|---|
| Munich | 17 November 2024 | Celik, Hasan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 7209

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2024

| Patent document cited in search report | | Publication date | Patent family member(s) | | Publication date |
|---|---|---|---|---|---|
| US 2007177713 | A1 | 02-08-2007 | CN | 1918605 A | 21-02-2007 |
| | | | EP | 1719082 A1 | 08-11-2006 |
| | | | JP | 2007521906 A | 09-08-2007 |
| | | | US | 2007177713 A1 | 02-08-2007 |
| | | | WO | 2005078664 A1 | 25-08-2005 |
| US 2012207370 | A1 | 16-08-2012 | NONE | | |
| WO 9733516 | A1 | 18-09-1997 | BR | 9702090 A | 02-02-1999 |
| | | | DE | 19780211 T1 | 08-07-1999 |
| | | | JP | H11505462 A | 21-05-1999 |
| | | | KR | 19990014762 A | 25-02-1999 |
| | | | NL | 1005515 C2 | 20-07-1998 |
| | | | US | 5671263 A | 23-09-1997 |
| | | | WO | 9733516 A1 | 18-09-1997 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **POLUDNIOWSKI, G. et al.** *Phys. Med. Biol.*, 2011, vol. 56, 1837

- **MASON, J. et al.** *arXiv:1706.08743, arXiv:1805.04411*